# EUROPEAN PATENT SPECIFICATION

(11) **EP 0 374 165 B1**
(45) Date of publication and mention of the grant of the patent: **02.09.1998**
(21) Application number: 88906723.7
(22) Date of filing: 20.07.1988
(51) Int. Cl.: C12N 5/06, C12M 3/02

(54) **AIRLIFT INSECT CELL CULTURE**
ZÜCHTUNG VON INSEKTENZELLEN MIT AIRLIFT-REAKTOREN
CULTURE DE CELLULES D'INSECTES PORTEES PAR L'AIR

(30) Priority: 24.07.1987 US 77181
(43) Date of publication of application: 27.06.1990
(73) Proprietor: CHIRON CORPORATION, Emeryville, California 94608 (US)
(72) Inventor: MAIORELLA, Brian, Oakland, CA 94618 (US); INLOW, Duane, Oakland, CA 94610 (US); HARANO, David, Oakland, CA 94619 (US)
(74) Representative: Bizley, Richard Edward
(86) International application number: US8802444
(87) International publication number: WO8901029

(56) References cited:
- Radlett,P.J.et al.,Applied Microbiology,1971,pp.534-537
- Miltenburger,H.G. et al, in 3rd general Meeting of ESACT, Oxford, 1979, Develop. biol. Standard. vol. 46, pp. 183-186
- Pollard R. et al., Process, Biochemistry, 1978, pp 47-55
- Luckov, V.A. et al., Bio/technology, January 88, pp 47-55
- Wu,J. et al., Appl. Microbiol Biotechnol. 1989, vol. 32, pp 249-225

## Description

This invention is in the field of fermentation and insect cell culture. This invention is particularly addressed to sparged insect culture and more particularly to airlift insect cell culture. Further, this invention provides for large scale in vitro growth of insect cells which are hosts for the production of viral and recombinant products. Still further, this invention concerns the expression of recombinant or viral products by insect cells grown in said airlift culture, that are infected respectively by a recombinant baculovirus or by a wild-type virus.

Airlift culture provides a means of increasing the surface/volume ratios of oxygen-containing gas to cell culture liquid in fermenting processes. Airlift fermentation principles are described in several reviews, for example, Onken et al., "Airlift Fermentors: Construction, Behavior, and Uses," Advances in Biotechnological Processes, 1:67-95 (1983); and Smart, "Gaslift Fermenters: Theory and Practice," Laboratory Practice (July, 1984).

Although the cell culture literature contains references concerning cultivation of microbial and mammalian cells by airlift culture, there have been no reports of successful cultivation of insect cells by airlift culture methods. Tramper et al., Enzyme Microb. Technol., 8:33-36 (January 1986) is exemplary wherein it is noted at p. 33: "A major problem encountered in scaling up insect cell culture systems is the shear sensitivity of these cells due to their size (20 µm range) and lack of cell wall. The shear sensitivity may hamper the supply of sufficient oxygen in a conventional manner (e.g., by sparging)." Tramper et al. further states at pages 35-36:
The mechanical strength of insect cells in culture is small.... This has definite consequences for the scale up of insect cell cultures. Larger volumes of insect cell cultures require more efficient oxygen transfer to the solution than can be achieved by flushing air/oxygen over the liquid surface. However, dispersion of gas by means of stirring and sparging air through the cell suspension to provide provide sufficient oxygen probably results in a larger decay than growth rate of the cells.

Tramper et al. reports that they were repeatedly unable to grow cells from Spodoptera frugiperda pupal ovaries maintained in a medium containing 10% fetal bovine serum and 0.02% silicon antifoam in an airlift reactor. "Apparently, rising and bursting of bubbles and not fluid velocity disintegrate the cells faster than they are able to grow in such a bioreactor." Tramper et al., p. 35

Weiss et al., In Granados et al. (eds.), The Biology of Baculoviruses, Vol. II, Chapter 3, pp. 63-87 (CRC Press, 1986), note at page 80: "Two problems appear to have delayed the full utilization of suspension systems for the large volume culture of insect cells: the fragility of insect cells... and second, the high oxygen demand, particularly for virus-infected cells...." Emphasis added; citations omitted as indicated by ellipses are respectively: Weiss et al., In Vitro, 16:222 (1980); and Hink et al., In Kurstak et al. (eds.), Invert. Tissue Culture: Applications in Medicine, Biology, and Agriculture, 297 (Academic Press 1976). The instant invention solves these problems.

Because of the literature perceived "fragility of insect cells," poorly aerated conditions have been conventionally used in insect cell culture, limiting scale up. Insect cells have been conventionally cultured in vessels, such as, spinner flasks or slowly stirred vessels, which rely only on above-surface gassing for aeration of the medium. In such conventionally used vessels, the ratio of liquid surface area to volume decreases as the vessel volume increases. Therefore, although the total oxygen demand of the insect cell culture increases in proportion to the increase in the volume of the culture, the capacity for oxygen transfer from above-surface gassing increases only in proportion to the liquid surface area. Oxygen starvation of insect cells thus limits the size of conventional insect culture vessels. The present invention overcomes such an oxygen transfer limitation and consequently the size limitations of insect cell culture.

Well-aerated conditions are necessary for optimal large scale growth of insect cells. Airlift culture is a means for providing the requisite oxygen transfer for such large scale growth.

Further, there remains a need in the art, as indicated above, for methods of growing insect cells in airlift culture without damaging the cells. The present invention meets such a need by providing methods of growing insect cells at large scale to high cell densities with high viability in sparged culture, particularly in airlift fermentors.

Insect cells have been successfully used to replicate recombinant baculoviruses to promote the expression of foreign genes carried thereby. Smith et al., PNAS (USA), 82:8404-8408 (1985); European Patent Application Publication No. 127,839 (published Dec. 12, 1984); and Jeang et al., J. Virol., 61(3):709-713 (March 1987). Insect cells have also been cultured for the production of insect viruses used as biological pesticides. Vaughn et al., In Vitro, 13:213-217 (1977); Lynn et al., J. Invert. Pathol., 32:1-5 (1978). Such viruses include, for example, baculoviruses and non-baculoviruses such as infectious flacheriae virus (IFV) and cytoplasmic polyhedrosis virus (CPV). Exemplary are certain baculoviruses, for example, nucleopolyhedrosis viruses (NPV) and granulosis viruses (GV), which are highly virulent for pest insects; some of the most promising have been commercially developed as biological pesticides pathogenic for agriculturally important insects. Burges (ed.), Microbial Control of Pests and Plant Diseases 1970-1980 (London, 1981); Miltenburger et al., Bioinsecticides II: Baculoviridae. Adv. Biotechnol. Processes, 3:291 (1984); for a discussion of such NPV and GV products as biological pesticides, see Shieh et al., "Production and Efficiency of Baculoviruses," Biotechnology and Bioengineering Vol. XXII, 1357 (1980); see also Huber, "Use of Baculoviruses in Pest Management Programs," In Granados et al., (eds.), The Biology of Baculoviruses: Vol. II Practical Applications for Insect Control, pp. 181-202 (1986).

Traditionally, production of baculoviruses was achieved using insect larvae; however, large scale production by such means is not very attractive. Insect cell culture is much more practical. Vaughn, Adv. Cell. Cult., 1:281-295 (1981); Stockton et al., In Burges (ed.), supra, at 313-328. Batchwise and semicontinuous production of Spodoptera frugiperda and Trichoplusia ni cells that allow the replication of Autographa californica nuclear polyhedrosis virus have been reported. Vaughn, J. Invert. Path., 28:233-237 (1976); Hink, In Kurstak (ed.), Microbial Viral Pesticides at 493-506 (1982).

The instant invention provides a means not only to grow insect cells at large scale to high cell density with high viability, but also to support the production therefrom of recombinant products, when the cells are infected with recombinant baculoviruses, or of other viral products, when the cells are infected with wild-type viruses.

The present invention provides for methods of growing insect cells in sparged culture, particularly in airlift fermentors. Such sparged or airlift culture overcomes the oxygen limitation which occurs in conventional insect culture vessels, that is, spinner flasks or slowly stirred vessels, which rely on above surface gassing for aeration of the medium. The present invention overcomes the size limitations of conventional insect cell culture by providing a new means of successfully aerating insect cultures by sparging and thereby overcoming the surface/volume ratio limitations inherent in above-surface gassing for oxygen transfer to the culture. The present invention allows insect cells to grow to high cell densities with high viability at a large scale.

In one aspect the invention provides a sparged method of cultivating insect cells to high cell density with high viability, wherein the culturc medium is sparged with gas, optionally at a sparging rate of from about 0.01 to about 0.08 vvm, and the culture medium comprises a polyoxypropylene polyoxyethylene condensate.

In another aspect there is provided a method of cultivating insect cells to high cell density with very high viability, wherein the culture medium is sparged with oxygen under conditions selected to produce bubbles ranging in diameter from about 0.2 cm to about 2.0 cms and a dissolved oxygen content of about 1% to to 150% of air saturation and the culture medium comprises a polyoxypropylene polyoxyethylene condensate and optionally one or more other non-toxic protective agents.

Also included in the invention are media for sparged culture of insect cells which comprise insect cells, a basal medium and one or more protective agents including at least one polyoxypropylene polyoxyethylene condensate, and the use in a culture medium of a polyoxypropylene polyoxyethylene condensate as a sparged insect cell cultivation protective agent to protect against cell damage and death.

The present invention discloses, therefore, operating parameters, most notably sparging rates and bubble size diameters, appropriate for sparged cell culture. Optimal sparge rates are in a range of from about 0.01 to about 0.08 volume total gas flow per volume culture per minute (vvm), more preferably from about 0.02 to about 0.06 vvm. Optimal bubble diameters are from about 0.2 cm to about 2.0 cm, and more preferably from about 0.3 cm to about 1.5 cm.

The present invention further provides for media in which to grow airlift insect cell cultures wherein the media comprise a polyoxypropylene polyoxyethylene condensate, a non-toxic protective agent that minimizes cell damage and death from sparging, that is, from gas bubbles used to drive the agitation/oxygenation system. Additional preferred non-toxic protective agents usable in this invention are non-toxic water soluble polymers; further preferred are non-toxic, non-ionic polymeric detergents. The polyoxypropylene polyoxyethylene condensates used are effective in reducing foaming, especially when the cells are maintained in serum containing media, and thereby preventing the loss of cells from the free suspension into the foam layer and adherence of cells to the vessel wall above the liquid surface.

Further, the invention provides for methods and media for growing insect cells at large scale and for supporting the production therefrom of recombinant products, when the cells are infected by recombinant baculoviruses, and viral products, when the cells are infected with wild-type viruses.

Large scale culture is defined herein to mean cultures of a volume approximately from about 5 L to about 25,000 L.

Sparged cultures are herein defined as cultures wherein compressed air or oxygen-containing gas is introduced into the nutrient medium, wherein cells are grown, through a sparger, that is, through either perforations or nozzles through which the compressed air or gas is forced into the medium during the fermenting process.

Airlift culture is herein defined as a method of cultivating cells including the steps of introducing the cells into a nutrient solution in which they tend to settle, and maintaining the cells in a suspended state so as to prevent their settling by introducing an oxygen-containing gas into the solution. Apparati for carrying out airlift culture include a container for a nutrient solution, an arrangement for introducing the oxygen-containing gas into the container and operative for guiding the nutrient solution so that the solution circulates within the container wherein the motive force for such circulation is the oxygen-containing gas. The guide arrangement may be formed as a tubular member, as a substantially flat partition wall, or as an inner wall of a circumferentially complete tubular container. Said tubular member may be a draught tube through the base of which the oxygen-containing gas is introduced by sparging, causing the culture fluid to circulate upward through the draught tube (the upcomer liquid stream) and then downward through the annular space between the draught tube and the vessel wall (the downcomer liquid stream). A gentle circulatory flow is induced in that the upcomer stream containing bubbles of the oxygen-containing gas is less dense than the downcomer stream and is displaced thereby. Dissolved oxygen tension and pH can be controlled by varying the composition of the sparged gas.

As indicated above, sparging has been noted in the literature to cause cell damage and death attributed to the "rising and bursting of bubbles" Tramper et al., supra , associated with the art perceived "fragility of insect cells" Weiss et al., supra and the small "mechanical strength of insect cells in culture" Tramper et al., id. . The airlift culture methods of the instant invention protect insect cells from damage and death under the well-aerated conditions of sparged culture.

An aspect of this invention features the specification of sparging rates for insect culture. The sparging rate is selected to be adequate to maintain good cell suspension and adequate oxygenation, but not so high as to cause cell damage. A further criterion in selecting a sparge rate is to produce a concentration of bubbles in the culture fluid low enough such that bubble-to-bubble interactions are minimized--thus, minimizing bubble coalescence such that the bubble size can be controlled by choice of sparger orifice size without concern for increase in bubble size due to coalescence.

Spate rates are maintained according to this aspect of this invention from about 0.01 to about 0.08 volume total gas flow per volume culture per minute (vvm), and preferably from about 0.02 to about 0.06 vvm. The sparged gas can be air which can maintain an adequate cell density. However, to avoid cell density limitations, the sparged gas is preferably supplemented with pure oxygen. Thus, the sparged gas can comprise a mixture of oxygen and a non-toxic diluent gas. The dissolved oxygen concentration (DO) of the culture medium can be maintained at a concentration anywhere from about 1% to 150% of air saturation, by methods known to those of ordinary skill in the art, depending on the particular requirements appropriate for the insect cell line and parameters selected. In general, as oxygen can be toxic to cells at high concentrations, the DO is preferably maintained below 100% of air saturation, and more preferably about or below 20% of air saturation; however, such a statement is only a general guideline, and the DO for the particular cell line and parameters used should be maintained at the optimal level therefor.

According to another aspect of this invention, to reduce damage to the insect cells caused from bubbles introduced through sparging, the sparged bubbles range in diameter size from about 0.2 cm to about 2.0 cm, and, more preferably from about 0.3 cm to about 1.5 cm. Bubble size can be regulated by controlling the dimensions of the orifices of the sparger. In this aspect of the invention, the dissolved oxygen content is maintained at from 1% to 150% of air saturation.

A primary aspect of the methods of this invention is the use in a sparged method of cultivating insect cells of a culture medium sparged with gas and containing a polyoxypropylene polyoxyethylene condensate as a protective agent and optionally one or more other protective agents. The protective agent or agents acts or act to prevent a disintegration/clumping phenomenon of insect cells grown under sparged conditions and further prevent(s) their adherence to the vessel walls. Further, the protective agent reduces the amount of cellular debris in the culture indicating that cell lysis is reduced by the presence of the protective agent. As foaming can occur in airlift culture due to the effects of the sparged gas on certain elements of media used, for example, on serum proteins therein, preferably, the protective agent also acts as an anti-foaming agent preventing the loss of cells from the free suspension into the foam layer, and acts as a bubble surface tension reducing agent and/or as a cell surface stabilizing agent and/or as a viscosifying agent to prevent or reduce bubble damage. Foaming can also be a significant problem in airlift insect cell culture if a microcarrier system, rather than a suspension system, is used in that microcarriers tend to concentrate in the foam layer.

Protective agents are herein defined as non-toxic, water soluble compounds that functionally act to protect insect cells from damage and death under well-aerated culture conditions. The protective agents of this invention are preferably non-toxic, water soluble polymers. A protective agent candidate can be selected by first confirming that it is not toxic to the insect cells to be cultured by methods known to those skilled in the art of insect cell culture, for example, by adding it to a suspension or monolayer of the insect cells of choice for cultivation and comparing the growth of the culture to a control. Then, the non-toxic protective agent candidates can be tested for protective ability by adding the candidate agent to rapidly agitated or sparged culture of the insect cells of choice at small scale and observing viability after an appropriate period and comparing the viability of the cells of said culture to the viability of the cells in a control culture.

The general correlation between the effectiveness of a protective agent in both agitated and sparged cultures is helpful in simplifying the selection of a suitable non-toxic protective agent for the airlift insect culture media and methods of this invention. Whereas airlift culture could be considered impractical at culture volumes of less than 5 L, small shake flask cultures (a control and test culture) are good models for determining the protective ability of a candidate protective agent. Still further simplifying such determination is the use of the disintegration/clumping phenomenon as the standard criterion for protective ability. If disintegration and clumping of cells occurs in the control flask but not in the flask containing the candidate agent, the agent is considered to have protective ability. Example 1, infra, provides a model system for such a method of selecting protective agents of this invention.

The media of this invention include as a protective agent a polyoxypropylene polyoxyethylene condensate and optionally one or more other protective agents which are preferably cell surface stabilizing agents and/or viscosifying agents and/or bubble surface tension reducing agents. Examples of such other protective agents are hydroxyethyl starch, methyl cellulose, carboxymethyl cellulose (as, sodium carboxymethyl cellulose), dextran sulfate, polyvinylpyrrolidone, ficoll, alginic acid, polypropyleneglycol, and non-toxic polymeric detergents.

Non-toxic polymeric detergents are preferred as any additional protective agents in the methods of this invention. Further preferred are non-toxic polymeric detergents which are non-ionic. Editions of McCutcheon's Emulsifiers & Detergents (published by the McCutcheon Division of MC Publishing Co, 175 Rock Road, Glenn Rock, N J, USA) are examples of a source of finding non-toxic, non-ionic polymeric detergent candidates for protective agents for the media of this invention, which can be tested for non-toxicity, and protective ability as indicated above. One class of non-toxic, non-ionic polymeric detergents are block copolymers of propylene oxide and ethylene oxide (polyoxypropylene polyoxyethylene condensates), preferably Pluronic polyols, such as, Pluronic F68, F77, F88 and F108, preferably F68 and F88, more preferably F68. Such pluronic polyols, which are essential to the invention, are useful because of their anti-foaming ability. The Pluronic polyols are commercially available from BASF Wyandotte Corp. (101 Cherry Hill Road, PO Box 181, Parsippany, N J 07054, USA).

The protective agent is preferably present in the media of this invention at a concentration which is most effective in protecting the insect cells from damage, but which concentration is non-inhibitory to cell growth and reproduction. The Pluronic polyol polymeric protectants are present in the media of this invention preferably at a concentration (weight-volume) of from about .01% to about 1%, more preferably from about .05% to about 0.5%, and still more preferably about 0.1%.

A still further preferred feature of the methods of this invention to protect insect cells from damage caused by foaming, that can occur in airlift culture, concerns the adjustment of the liquid height above the draught tube to find an optimal level where the culture liquid flow can be used as a foam breaker. Foam height was found to be a function of liquid height above the draught tube. In media containing high concentrations of proteins, as, for example, in media that are supplemented with 9% serum, foam height is minimized when the liquid height is adjusted such that the circumferential area of the cylindrical section above the draught tube (that is, the cylindrical section corresponding in diameter to the draught tube that extends from the top of the draught tube to the surface of the liquid in the fermentor) is preferably from about 1.5 to about 2.5 times, and more preferably about 2 times, the horizontal cross-sectional area of the downcomer annulus. Said circumferential area is calculated as πdh wherein "d" is the diameter of the draught tube and "h" is the height of the liquid above the draught tube, that is, the difference between the surface of the liquid and the top of the draught tube.

In serum-free, low or no protein media, as discussed below, foam height is minimized by adjusting the liquid height such that the circumferential area of the cylindrical section above the draught tube is preferably from about 2.5 to about 5.5 times, and more preferably about 4.5 times, the horizontal cross-sectional area of the downcomer annulus.

It is further preferred according to the methods of this invention that the horizontal cross-sectional area of the upcomer section (that is of the draught tube per se is from about 1 to about 1.5 times the horizontal cross-sectional area of the downcomer annulus.

To maintain a relatively stable oxygen concentration throughout the airlift culture, the dimensions of the vessel, at the preferred sparging rates herein defined, are preferably such that the ratio of the height of the vessel to its diameter is in the range of from about 3/1 to about 12/1, and more preferably from about 6/1 to about 7/1. At such dimensions, an environment is created to maximize cell viability and density in that the oxygen concentration is not depleted within the downcomer annulus and adequate agitation and oxygen transfer at a preferred sparging rate is maintained throughout the vessel.

Insect cells can be grown by the airlift culture methods of this invention in any media which provide a good nutritional environment, and comprise a non-toxic protective agent as described above. A "basal medium" is herein defined as a nutrient mixture of inorganic salts, sugars, amino acids, optionally also containing vitamins, organic acids and/or buffers. Basal media together with supplements provide the nutrients necessary to support cell life, growth and reproduction. The basal media can be supplemented or not supplemented with serum and proteins, such as, albumin.

If the media are not supplemented with serum and proteins, such media are referred to as serum free and contain no or very little protein, and preferably comprise (1) a basal medium; (2) a lipid/emulsifier component; (3) a peptone component; and (4) a protective agent or agents under well-aerated conditions as in sparged cultures as those described herein.

The peptone component is preferably ultrafiltered to remove any residual proteases, high molecular weight components , or endotoxins. The peptone component of such serum free media can be selected from a wide variety of hydrolyzed protein products, either alone or in combination, but are preferably yeast extract, more preferably Yeastolate (Difco, USA) alone or in combination with Lactalbumin Hydrolyzate (LH), at a concentration from about 1 g/L to about 12 g/L, preferably from about 2 g/L to about 8 g/L, and more preferably from about 3 g/L to about 5 g/L. Still more preferably, the peptone component comprises Yeastolate alone at a concentration of about 4 g/L or Yeastolate and LH in combination each at a concentration of about 2 g/L.

The lipid/emulsifier component is preferably supplied to the media in the form of a microemulsion. The lipid/emulsifier component preferably comprises lipids essential for the growth of insect cells and are preferably selected from the group comprising a mixture of polyunsaturated fatty acid esters, preferably methyl esters, and more preferably cod liver oil, preferably at concentration of from about 1 mg/L to about 50 mg/L lipid soluble vitamins, preferably alpha-tocopherol, (preferably at a concentration of from about 0.5 mg/L to about 4 mg/L), and steroids, preferably sterols and more preferably cholesterol (preferably at a concentration of about 2 mg/L to about 7 mg/L). The emulsifier or emulsifiers present in the lipid/emulsifier component preferably include phospholipids, more preferably lecithin, and non-toxic, non-ionic polymeric detergents (preferably at a concentration from about 5 mg/L to about 75 mg/L), more preferably polysorbate compounds, and still more preferably polysorbate 80.

There are a wide variety of commercially available basal media that can be used in the media of this invention. Such commercially available basal media include, for example, TC10 without tryptose broth commercially available from Microbiological Associates; see Gardiner et al., J. Invert. Pathol., 25:363 (1975), Grace's Antheraea medium Vaughn et al., TCA Manual, 3(1) (1976); Yunker et al., Science, 155:1565-1566 (1967) , Medium M20 of Mark's Vaughn et al., TCA Manual, 3 (1) 1976); Marks, In Kruse et al. (eds.), Tissue Culture Methods and Applications, pp. 153-156 (1973), Goodwin's IPL-52 Medium Goodwin, In Vitro, 11:369-378 (1975), Goodwin's IPL Medium Goodwin et al., In Kurstak et al. (eds.), Invertebrate Systems In Vitro (1980), Goodwin's IPL-76 Peptone Medium Goodwin et al., id.; Goodwin et al., In Vitro, 14:485-494 (1978), Hink's TMH FH Medium (Revised) Hink, Nature (London), 226:466-467 (1970) , Medium S-301 of Hansen Hansen, In Maramorosch (ed.), Invertebrate Tissue Culture Research Applications, pp. 75-99 (1976); Vaughn et al., TCA Manual, 3(1) (1976) , and IPL-41 Medium Weiss et al., In Vitro, 17 (6):495-502 (1981) , wherein IPL-41 is a preferred basal medium.

As indicated, IPL-41 is a preferred basal medium for the preparation of the media for this invention. IPL-41 basal medium is commercially available from a number of vendors and is described in Weiss et al., In Vitro, 17 (6):495-502 (June 1981) and in Weiss et al., CRC Press, supra, pp. 70-72 (1986). Table 1 of Weiss et al. (In Vitro) at page 496, and Table 3 of Weiss et al. CRC Press, at pages 71-72 outline all the components of IPL-41 and provide their proportions in mg/l; said tables are herein incorporated by reference. At page 497 of Weiss et al. (In Vitro), the preparation of the complete medium IPL-41 is described wherein tryptose phosphate broth (TPB) and fetal bovine serum (FBS) are added. The IPL-41 basal medium employed in preparing the media of this invention preferably do not contain tryptose phosphate broth (TPB), and more preferably contain as a replacement therefor, yeast extract, preferably Yeastolate (Difco) or Yeastolate and Lactalbumin Hydrolyzate at appropriate concentrations.

The media for the airlift culture of this invention can be inoculated with insect cells maintained in any variety of culture modes and conditions but preferably are inoculated with cells that are in an exponential growth phase, that is, cells that have been maintained under non-oxygen limited and non-nutrient limited conditions.

The media employed in the methods of this invention are preferably those which enhance cell growth and viability and support the production of viral and recombinant products from insect cells infected respectively by wild-type or recombinant viruses.

The insect cells grown according to the airlift culture methods of this invention are cultured in a temperature range and under conditions appropriate for the particular cell line selected. For example, Spodoptera frugiperda cells, that is Sf9 cells, are cultured in a temperature range of from about 25°C to about 32°C, preferably from about 27°C to about 28°C and wherein the pH of the culture medium is preferably maintained in a range of from about 6 to about 7.0, more preferably about 6.2 to about 6.4.

Insect cells that can be grown successfully by the airlift culture methods of this invention are those which grow successfully in agitated culture, such as, shake flasks, wherein the medium contains a protective agent or agents. Therefore, a simple test can be designed to determine whether a particular insect cell line can be grown successfully by the airlift culture methods of this invention wherein the candidate insect cells are tested for appropriate growth and viability criteria in a small shake flask culture wherein the medium contains a non-toxic protective agent as herein described.

Analogously, insect cells which can be grown successfully and which can produce viral products or recombinant proteins upon infection, respectively, with either wild-type viruses or recombinant viruses, are those which have been shown to grow, reproduce and express recombinant or viral products in agitated culture wherein the medium contains a non-toxic protective agent. Candidate insect cells grown for production of viral or recombinant products can be tested analogously as indicated above.

Candidate insect cells that can be grown according to the airlift culture methods of this invention can be from any order of the Class Insecta, preferably those which can be hosts to a baculovirus expression vector system, or other wild-type viruses. Preferably, the insect cells are selected from the Diptera or Lepidoptera orders. About 300 insect species have been reported to have nuclear polyhedrosis virus (NPV) diseases, the majority (243) of which were isolated from Lepidoptera. Weiss et al., "Cell Culture Methods for Large-Scale Propagation of Baculoviruses," In Granados et al. (eds.), The Biology of Baculoviruses: Vol. II Practical Application for Insect Control, pp. 63-87 at p. 64 (1986). Insect cell lines derived from the following insects are exemplary: Carpocapsa pomonella (preferably cell line CP-128); Trichoplusia ni (preferably cell line TN-368); Autographa californica; Sodoptera frugiperda (preferably cell line Sf9); Lymantria dispar; Mamestra brassicae; Aedes albopictus; Orgyia pseudotsugata; Neodiprion sertifer; Aedes aegypti; Antheraeaeucalypti; Gnorimoschema opercullela; Galleria mellonella; Spodoptera littolaris; Blatella germanica; Drosophila melanogaster; Heliothis zea; Sodoptera exigua; Rachiplusia ou; Plodia interpunctella; Amsaeta moorei; Agrotis c-nigrum, Adoxophyes orana, Agrotis segetum, Bombyx mori , Hyponomeuta malinellus, Colias eurytheme, Anticarsia germmetalia, Apanteles melanoscelus, Arctia caja, and Porthetria dispar. Preferred insect cell lines are from Spodoptera frugiperda, and especially preferred is cell line Sf9. The Sf9 cell line used in the examples herein was obtained from Max D. Summers (Texas A & M University, College Station, TX 77843 USA). Other S. frugiperda cell lines, such as IPL-Sf-21AE III, are described in Vaughn et al., In Vitro, 13:213-217 (1977).

The insect cell lines of this invention are preferably suitable for the reproduction of numerous insect-pathogenic viruses such as parvoviruses, pox viruses, baculoviruses and rhabdoviruses, of which nucleopolyhedrosis viruses (NPV) and granulosis viruses (GV) from the group of baculoviruses are preferred. Further preferred are NPV viruses such as those from Autographa spp., Spodoptera spp., Trichoplusia spp., Rachiplusia spp., Galleria spp. and Lymantria spp. More preferred are baculovirus strains Autographa californica NPV (AcNPV), Rachiplusia ou NPV, Galleria mellonella NPV and any plaque-purified strains of AcNPV, such as E2, R9, S1, M3, characterized and described by Smith et al., J. Virol., 30:828-838 (1979); Smith et al., J. Virol., 33:311-319 (1980); and Smith et al., Virol., 89:517-527 (1978).

European patent application 127,839 (published December 12, 1984) and U.S. Patent No. 4,745,051 to Smith et al. describe a method for producing a recombinant baculovirus expression vector, capable of expressing a selected gene in a host insect cell. The recombinant baculovirus expression vector is cotransfected with wild-type baculovirus DNA into a host insect cell, wherein recombination occurs. Recombinant baculoviruses are then detected and isolated according to methods described in EP 127,839 and Summers et al., "A Manual and Methods for Baculovirus Vectors and Insect Cell Culture Procedures" (January 17, 1986). The resultant recombinant baculovirus is then used to infect cultured insect cells and the protein product from the incorporated selected gene is expressed by the insect cells and secreted into the medium. Exemplified therein is the production of recombinant beta-interferon, interleukin-2, and chloramphenicol acetyltransferase (CAT) via the culturing of S. frugiperda cells infected with a recombinant AcNPV expression vector into the genome of which the appropriate gene had been inserted. Further information concerning such a recombinant baculovirus expression system and its use in expressing recombinant proteins can be tound in Summers et al., id..

European Patent Application 127,839, U.S. Patent No. 4,745,051 and Luckow, V.A. (1988) Biotechnol. 6:47-55 provide enablement concerning methods for producing recombinant baculovirus transfer vectors, and recombinant baculoviruses and for the use of the recombinant baculovirus expression system for expressing recombinant proteins in host insect cells. Said European application, U.S. patent and publication are herein incorporated by reference.

A specific example of the recombinant products that can be produced by the airlift culture methods of this invention is recombinant CSF-1 which can be produced by the host insect cells and infected with recombinant baculoviruses, such as AcM4 or AcM6, and cultured according to this invention. However, those skilled in the art who have the benefit of this disclosure, as well as the above publications incorporated by reference, will recognize that many other recombinant proteins can be produced by insect cells infected with recombinant baculovirus according to this invention. Other heterologous proteins that have been expressed in insect cells via the BEVS are outlined in Summers et al., "Genetic Engineering of the Genome of the Autographa californica nuclear polyhedrosis virus," Banbury Report:Genetically Altered Viruses in the Environment, 22:319-329 (1985) and in Miller, D.W. et al. (1986) in Genetic Engineering Principles and Methods Vol 8, pp. 277-298; Settow and Hollaender, eds. , Plenum Press, N.Y. Exemplary recombinant poteins include, without limitation, colony stimulating factors for example, long and short form CSF-1 (described below), G-CSF, GM-CSF among others interferons (alpha, beta and gamma and hybrids thereof), interleukins, tumor necrosis factor, erythropoietin, human growth hormone, as well as porcine, bovine and other growth hormones, epidermal growth factor, insulin, modified pro-urokinase or urokinase, tissue plasminogen activator (TPA), TPA-urokinase hybrids, hepatitis B vaccine, superoxide dismutase, Factor VIII, atrial natriuretic factor, feline leukemia virus vaccines, as, for example, gp70 polypeptides, toxic proteins such as whole ricin toxin, ricin A chain, products containing ricin A, other lectins such as Ricin communis agglutinin (RCA), diptheria toxin, gelonin, exotoxin from Pseudomonas aeruginosa, toxic proteins from Phytolacca americana (PAPI, PAPII and PAP-S) , insecticidal proteins from Bacillus thuringiensis, many enzymes as for example, CAT, as well as innumerable other hybrid proteins.

"Colony stimulating factor (CSF-1)" refers to a protein which exhibits the spectrum of activity understood in the art for CSF-1, that is, when applied to the standard in vitro colony stimulating assay of Metcalf, J. Cell Physiol., 76:89 (1970), it results in the formation of primarily macrophage colonies. Native CSF-1 is a glycosylated dimer; dimerization may be necessary for activity. The term CSF-1 herein refers to both dimeric and monomeric forms.

Human CSF-1 is operative both on human and murine bone marrow cells, whereas murine CSF-1 does not show activity with human cells. Therefore, human CSF-1 should be positive in the specific murine radioreceptor assay of Das et al., Blood, 58:630 (1981). The biological activity of the protein is also inhibited by neutralizing antiserum to human urinary CSF-1. Das et al., id.

CSF-1 is able to stimulate the secretion of series E prostaglandins, interleukin-1 and interferon from mature macrophages. Moore et al., Science, 223:178 (1984). However, the protein's ability to stimulate the formation of monocyte/macrophage colonies using bone marrow cells (bone marrow assay) and its susceptibility to inhibition by neutralizing antiserum against purified human urinary CSF-1 as well as a positive response to the radioreceptor assay (RRA) or a conventional radioimmunoassay (RIA) can be employed to identify CSF-1 produced by insect cells via a recombinant baculovirus expression vector system (BEVS).

As described in PCT Publication No. WO88/03173 published May 5, 1988, the production of biologically active CSF-1 is complicated by the high degree of post-translational processing which includes glycosylation and dimerization. As indicated in Luckow, V.A. et al. (supra) , it is clear that the colony stimulating factors are secreted into the medium. Molecular weights of the CSF proteins produced indicate that the signal peptide is cleaved. The products also appear to be glycosylated.

Various forms of CSF-1, including a short form and a long form, have been described. See Kawasaki et al., Science, 230:291-296 (October 18, 1985); Ladner, M.B. et al., EMBO J. 6:2693-2698 (1987); Wong et al., Science, 235:1504-1508 (March 20, 1987); Clark et al., Science, 236:1229-1237 (June 5, 1987); Metcalf, supra. Recombinant CSF-1 as well as muteins corresponding to the cDNA encoded forms are disclosed and claimed in PCT Publication Nos. WO86/04607 published August 14, 1986, WO88/03173 published May 5, 1988 and WO87/06954 published November 19, 1987.

Baculovirus transfer vectors containing CSF-1 genes, pAcM4 and pAcM6, were used to prepare the recombinant baculoviruses AcM4 and AcM6, by cotransfection with baculovirus DNA in Sf9 cells. Said recombinant baculovirus transfer vectors pAcM4 and pAcM6 in E. coli/MM294 have been deposited at the American Type Culture Collection (ATCC), 12301 Parklawn Drive, Rockville, MD 20852 (USA) on June 12, 1987 respectively under the designation ATCC Nos. 67428 and 67429. said vectors have also been deposited and are maintained in the Cetus Master Culture Collection (CMCC) under the respective designations CMCC No. 3002 and CMCC No. 2996.

Recombinant baculovirus AcM4 carries a nucleotide sequence which encodes a 150 amino acid form of rCSF-1 whereas the recombinant baculovirus AcM6 carries a nucleotide sequence which encodes a 522 amino acid form of rCSF-1.

pAcM4 is an in frame translational fusion vector constructed by cloning a mutated CSF-1 cDNA fragment into pAcC1. pAcC1 is similar to pAc401 (described in Luckow, V.A. et al., supra) except that the recognition site for EcoRI endonuclease has been removed. To accomplish this, pAc401 was digested to completion with EcoRI and the ends were made blunt using Klenow fragment. After ligation and transformation, candidates were screened for the absence of an EcoRI site. The source of the CSF-1 cDNA fragment was pCSF-gly150 described in PCT Publication No. WO86/04607 which contains a TGA stop codon instead of histidine codon at position 151. In addition, a SmaI recognition site was introduced 3 bp after the ATG translational start codon of the CSF-gly150 coding sequence by site-specific mutagenesis.

The desired construct pAcM4 thus contains the intact polyhedrin gene protomer and 5' untranslated leader but three altered codons in the CSF-1 signal peptide. Instead of Met-Thr-Ala-Pro-Gly...the sequence in this construct encodes Met-Arg-Pro-gly-Gly... The CSF-1 5' untranslated leader sequence has been removed so that all of the sequence 5' to the ATG translational start is derived from the AcNPV polyhedrin gene.

pAcM6 was constructed by excising the 250 pb StuI (positions 511-821 pb on the pcCSF-17 sequence) fragment from pAcM4 (described in Section B.3) and replacing it with the 1141 bp StuI (positions 343-1484 bp on the CSF-4 sequence) fragment from pcDBhuCSF-4. The 1141 bp StuI fragment from pcDBhuCSF-4 contains the sequence from within amino acid 101 to within amino acid 184 of the shorter pcCSF-17 CSF sequence, with an additional coding sequence for 298 amino acids inserted after amino acid 149. Said in another way, the resulting construction, pAcM6, has the same 5' and 3' sequence as found in pAcM4 with the additional 894 bp of additional coding sequence form pcDBhuCSF-4 inserted within the CSF-1 coding sequence.

The effect of timing of the infection of the insect cells with a recombinant baculovirus has been shown to be critical for enhanced specific productivity. The specific production of the recombinant protein was found to be constant during the exponential phase of cell growth under non-oxygen limited conditions. Late infection, under non-exponential growth conditions, resulted in lower specific productivity and lower final titer. It is preferred that the exponential growth phase be extended to the highest possible cell densities to achieve the highest total productivity of the recombinant protein product. Infection of the host insect cells under conditions that limit growth, for example, in the stationary phase of cell growth, results in a reduced specific productivity of the recombinant protein product.

Specific productivity of the recombinant protein product is relatively independent of cell density at the time of infection as long as the culture is in exponential growth. For example, when Spodoptera frugiperda cells are the host insect cells, preferred cell densities of from about 1.0 to about 4.0 x 10⁶ cells/ml are preferred for infection with the recombinant baculovirus, more preferably from about 2.5 to about 3.5 x 10⁶ cells/ml.

The timing of the harvest of the recombinant protein product is critical to avoid contamination of the recombinant protein by viral and cell lysis proteins and to simplify thereby the downstream purification of the recombinant product. With considerations for the stability of the product, it would be preferred to harvest the recombinant product before significant cell lysis has occurred. Further, each recombinant protein or viral product to be produced according to the methods and media of this invention should be checked for stability and degradation over the course of the fermentation run. Such considerations should enter into a determination of the optimal harvest time.

The following examples further illustrate the airlift culture methods of this invention. These examples are not intended to limit the invention in any manner.

### EXAMPLE 1

This example provides a model small scale shake flask culture method for selecting appropriate protective agents for the airlift culture media and methods of this invention. The particular parameters described in this example may not be appropriate for all insect cell lines. For a particular insect cell line, conditions should be found whereby agitation is sufficient to cause within one or two days a disintegration/clumping phenomenon in a control culture without a protective agent.

Two 100 ml cultures of Sf9 inoculated at 1 x 10⁵ cells/ml were grown in 250 ml shake flasks agitated at 100-150 rpm (with an orbital radius of one-half inch) at approximately 27°C. One culture, the control culture, was maintained in IPL-41 basal medium supplemented with 9.1% fetal bovine serum and 4 g/L Yeastolate (Difco); whereas, the test culture was maintained in a medium corresponding to the control but with the protective agent, Pluronic F68 at 0.1% (weight-volume) concentration.

The two cultures were then observed for growth and viability. After about 36 hours, the control culture evidenced the disintegration/clumping phenomenon; the cells therein were not growing but dying as determined by Trypan blue exclusion. The test culture with the protective agent grew well with greater than 99% viability and by the fifth day had reached a cell density of about 5 x 10⁶ cells/ml.

### EXAMPLE 2

This example demonstrates that Sodoptera frugiperda cells (Sf9) were successfully grown in a 25 liter airlift fermentor from 9 x 10⁴ cells/ml up to 5 x 10⁶ cells/ml. By contrast, a 2.4 L spinner reached a cell density of only 1.5 x 10⁶ cells/ml. The Sf9 airlift culture grew with a doubling time (Td) of from 23-29 hours, and with viability in excess of 97%.

A static culture of Sf9 cells was transferred into, and maintained in, IPL-41 complete medium with, that is, tryptose phosphate broth (2.6 g/L) and 9.1% heat inactivated fetal bovine serum, to which was added 0.1% Pluronic polyol (Pluronic F68). This medium was used for all the experiments recorded in this example. Suspension cultures were maintained in spinner flasks at 60 rpm at room temperature (26-32°C. Growth was monitored with a Coulter Counter. Cell viability was determined with Trypan Blue vital stain and microscopic counting. In most cases, viability was 99+%. The cell densities referred to herein are viable cell counts.

A 100 ml spinner flask (Bellco Catalog #196500100) with a surface/volume ratio of 0.25 cm²/ml was inoculated at 1 x 10⁵ cells/ml with Sf9 cells that had been resuspended from a 6 day old static culture. The culture volume was reduced from 100 ml to 50 ml after 96 hours of growth (1.4 x 10⁶ cells/ml ) thereby doubling the surface/volume ratio.

A 2.4 L culture was grown in a 3 liter spinner flask (Bellco Catalog #196503000) with a surface/volume ratio of about 0.084 cm²/ml. The initial cell density was 1.4 x 10⁵ cells/ml inoculated from a 500 ml spinner in mid-exponential phase of growth.

A 25 liter Chemap airlift fermentor (Catalog No. 91001674-06) was inoculated at 9 x 10⁴ cells/ml with Sf9 cells that had been grown to late exponential phase (9.3 x 10⁵ cells/ml) in a 3 liter spinner. The initial culture volume was 22 liters. The fermentor was operated at 28°C. Agitation was maintained by sparging with nitrogen and oxygen at 0.02 volume total gas flow per volume culture per minute (about 0.4 liters per minute). Dissolved oxygen was maintained at approximately 20% air saturation by controlling the concentration of oxygen in the sparge gas.

The growth curve for the 100 ml spinner culture indicated that cell density plateaued at over 5 x 10⁶ cells/ml with 97-99% viability. This small spinner was assumed to provide relatively good oxygen transfer properties due to the low volume to surface area ratios therein. Cell growth was nearly exponential up to 2.7 x 10⁶ cells/ml. The population doubling time (Td) was 19-25 hours. There was a halt in cell growth at 5.3 x 10⁶ cells/ml, which was attributed to a depletion of nutrients.

The growth curve for the 2.4 liter spinner culture indicated that growth was nearly exponential up to 5 x 10⁵ cells/ml with a population doubling time of 24-28 hours. Cell growth became linear above 8 x 10⁵ cells/ml. The culture viability dropped significantly before the culture reached 1.5 x 10⁶ total cells/ml, and the peak viable cell density almost reached 1.2 x 10⁶ cells/ml. A linear (versus exponential) increase in cell density is diagnostic of oxygen limitation. The poor oxygen transfer associated with the increased culture volume was considered the reason for the poor performance of the 2.4 liter spinner culture as compared to the 100 ml spinner culture.

The growth curve of the 25 liter Chemap airlift fermentor indicated that cell growth was similar to that found in the 100 ml spinner culture. The cell density peaked at about 5 x 10⁶ cells/ml with 97% viability. The exponential phase of growth up to 1 x 10⁶ cells/ml had a Td of 23 hours, followed by a Td of 29 hours up to 3.6 x 10⁶ cells/ml. The cell density increased until levels similar to those seen in the small-scale 100 milliliter spinner were reached.

As indicated above, both the small spinner (100 ml) culture and the airlift fermentor culture had peak cell densities of about 5 x 10⁶ cells/ml with 97-99% viability. Whereas the 2.4 liter spinner culture reached a viable cell density of only 1.2 x 10⁶ cells/ml.

### Conclusion

In summary, it can be seen that the airlift culture methods and media of this invention allow insect cells to grow in sparged culture, particularly in airlift fermentors, to high cell densities with high viability at a large scale. Further, the methods herein provide the means to scale-up insect cell culture to produce successfully viral and recombinant products from the culture when tne cells are infected by either wild-type or recombinant viruses.

### Deposit

As mentioned above, the recombinant baculovirus transfer vectors pAcM4 and pAcM6 in E. coli/MM294 were deposited on June 12, 1987 at the American Type Culture Collection (ATCC), 12301 Parklawn Drive, Rockville, MD 20852 (USA) respectively under the designation of ATCC Nos. 67429 and 67428.

Said deposits were made pursuant to a contract between the ATCC and the assignee of this patent application, Cetus Corporation. The contract with the ATCC provides for permanent availability of said strains and progeny thereof to the public upon issuance of a U.S. patent related to this application describing and identifying the deposits or upon the publication or laying open to the public of any U.S. or foreign patent application, whichever comes first, and for the availability of the strain and the progeny thereof to one determined by the U.S. Commissioner of Patents and Trademarks to be entitled thereto according to 35 USC .122 and the Commissioner's rules pursuant thereto (including 37 CFR .1.14 with particular reference to 886 OG 638). The assignee of the present application has agreed that if the strains on deposit should die or be lost or destroyed when cultivated under suitable conditions, it will be promptly replaced upon notification with a viable culture of the same strains.

The deposits under the terms of the Budapest Treaty assure that said cultures deposited will be maintained in a viable and uncontaminated condition for a period of at least five years after the most recent request for the furnishing of a sample of the deposited microorganism was received by the ATCC and, in any case, for a period of at least 30 years after the date of the deposit.

Availability of the deposited strains are not to be construed as a license to practice the invention in contravention of the rights granted under the authority of any government in accordance with its patent laws.

Also, the present invention is not to be considered limited in scope by the deposited recombinant transfer vectors, since the deposited vectors are intended only to be illustrative of particular aspects of the invention. Any recombinant baculovirus transfer vector which can be used to prepare recombinant baculoviruses which can function to infect a host insect cell to produce a recombinant protein product is considered to be within the scope of this invention. Further, various modifications of the invention in addition to those shown and described herein will be apparent to those skilled in the art from the preceding description.

## Claims

1. A sparged method of cultivating insect cells to high cell density with high viability, wherein the culture medium is sparged with gas and the culture medium comprises a polyoxypropylene polyoxyethylene condensate.

2. A method according to claim 1 wherein the ratio of the height of the sparged vessel employed to its diameter is in the range of from about 3/1 to about 12/1.

3. A method according to claim 1 or claim 2 that supports the growth and reproduction of insect cells and the production therefrom of viral and recombinant products when the cells are infected with either a wild-type or recombinant baculovirus.

4. A method according to any of claims 1 to 3, wherein the culture medium is sparged at a rate of from about 0.01 to 0.08 vvm.

5. A method of cultivating insect cells to high cell density with very high viability, wherein the culture medium is sparged with oxygen under conditions selected to produce bubbles ranging in diameter from about 0.2 cm to about 2.0 cms and a dissolved oxygen content of about 1% to 150% of air saturation and the culture medium comprises a polyoxypropylene polyoxyethylene condensate and optionally one or more other non-toxic protective agents.

6. A method according to claim 5 which is further defined by the feature(s) of claim 2, claim 3 and/or claim 4.

7. Media for airlift culture of insect cells, which comprise insect cells, a basal medium and one or more protective agents including at least one polyoxypropylene polyoxyethylene condensate.

8. Media according to claim 7 which are substantially free from serum components and which further comprise a lipid component, and a peptone component.

9. The use of a culture of insect cells resulting from a method of cultivation according to any one of claims 1 to 6 in the expression of a recombinant or viral product.

10. The use in a culture medium of a polyoxypropylene polyoxyethylene condensate as a sparged insect cell cultivation protective agent to protect against cell damage and death.

11. The use of claim 10 which further includes the features of claim 7 and/or claim 8.

12. The use of claim 10 or claim 11, wherein the culture medium is an airlift culture medium.

13. A process of expressing a recombinant or viral product using insect cells, which comprises the use of a polyoxypropylene polyoxyethylene condensate as a protective agent in sparged culture medium-used for cultivating the cells, the process optionally further comprising the specific feature(s) of one or more of claims 2 to 6.

14. A process of claim 13, wherein the cells are cultivated by airlift culture.

## Patentansprüche

1. Durchblasverfahren zur Züchtung von Insektenzellen mit hoher Zelldichte und hoher Lebensfähigkeit, wobei durch das Kulturmedium Gas hindurchgeblasen wird und das Kulturmedium ein Polyoxypropylen-Polyoxyethylen-Kondensat umfaßt.

2. Verfahren nach Anspruch 1, wobei das Verhältnis der Höhe des verwendeten Gefäßes, durch das Gas hindurchgeblasen wird, zu seinem Durchmesser im Bereich von etwa 3/1 bis etwa 12/1 liegt.

3. Verfahren nach Anspruch 1 oder 2, das das Wachstum und die Vermehrung von Insektenzellen und deren Produktion von viralen und rekombinanten Produkten unterstützt, wenn die Zellen mit entweder einem Wildtyp-Baculovirus oder einem rekombinanten Baculovirus infiziert sind.

4. Verfahren nach einem der Ansprüche 1 bis 3, wobei durch das Kulturmedium Gas mit einer Rate von etwa 0,01 bis 0,08 VVM hindurchgeblasen wird.

5. Verfabren zur Züchtung von Insektenzellen mit hoher Zelldichte und sehr hoher Lebensfähigkeit, wobei durch das Kulturmedium Sauerstoff unter Bedingungen hindurchgeblasen wird, die so ausgewählt werden, daß Blasen mit einem Durchmesser im Bereich von etwa 0,2 cm bis etwa 2 cm und ein Gehalt an gelöstem Sauerstoff von etwa 1 % bis 150% Luftsättigung erzeugt werden, und das Kulturmedium ein Polyoxypropylen-Polyoxyethylen-Kondensat und gegebenenfalls ein oder mehrere weitere nichttoxische Schutzmittel umfaßt.

6. Verfahren nach Anspruch 5, das weiter durch das (die) Merkmal(e) von Anspruch 2, 3 und/oder 4 definiert ist.

7. Medien zur Airlift-Kultur von Insektenzellen, die Insektenzellen, ein Basalmedium und ein oder mehrere Schutzmittel, einschließlich mindestens eines Polyoxypropylen-Polyoxyethylen-Kondensats, umfassen.

8. Medien nach Anspruch 7, die im wesentlichen frei von Serumbestandteilen sind und weiter einen Lipidbestandteil und einen Peptonbestandteil umfassen.

9. Verwendung einer Kultur von Insektenzellen, die aus einem Züchtungsverfahren nach einem der Ansprüche 1 bis 6 erhalten werden, zur Expression eines rekombinanten oder viralen Produkts.

10. Verwendung eines Polyoxypropylen-Polyoxyethylen-Kondensats als Schutzmittel bei der Züchtung von Insektenzellen unter Hindurchblasen von Gas zum Schutz gegen Zellschådigung und Zelltod in einem Kulturmedium.

11. Verwendung nach Anspruch 10, die weiter die Merkmale von Anspruch 7 und/oder Anspruch 8 einschließt.

12. Verwendung nach Anspruch 10 oder Anspruch 11, wobei das Kulturmedium ein Airlift-Kulturmedium ist.

13. Verfahren zur Expression eines rekombinanten oder viralen Produkts unter Verwendung von Insektenzellen, umfassend die Verwendung eines Polyoxypropylen-Polyoxyethylen-Kondensats als ein Schutzmittel in einem zur Züchtung der zellen verwendeten Kulturmedium, durch das Gas hindurchgeblasen wird, wobei das Verfahren gegebenenfalls weiter das (die) spezifische(n) Merkmal(e) von einem oder mehreren der Ansprüche 2 bis 6 umfaßt.

14. Verfahren nach Anspruch 13, wobei die Zellen mittels Airlift-Kultur gezüchtet werden.

## Revendications

1. Procédé de culture par aspersion de cellules d'insectes à haute densité cellulaire avec un taux de viabilité élevé, selon lequel le milieu de culture est aspergé avec un gaz et ledit milieu de culture comprend un condensat de polyoxypropylène/polyoxyéthylène.

2. Procédé selon la revendication 1 selon lequel le rapport hauteur sur diamètre du récipient d'aspersion est compris entre environ 3/1 et environ 12/1.

3. Procédé selon la revendication 1 ou la revendication 2 qui permet la croissance et la reproduction de cellules d'insectes et la production à partir de celles-ci de produits viraux et recombinants lorsque les cellules sont infectées avec un baculovirus soit sauvage soit recombinant.

4. Procédé selon l'une quelconque des revendications 1 à 3, selon lequel le milieu de culture est aspergé à un taux d'environ 0,01 à 0,08 vvm.

5. Procédé de culture de cellules d'insectes à densité cellulaire élevée avec un taux de viabilité élevé selon lequel le milieu de culture est aspergé avec de l'oxygène sous des conditions sélectionnées de manière à produire des bulles dont le diamètre varie d'environ 0,2 cm à environ 2,0 cm et la teneur en oxygène dissous est d'environ 1 % à 150 % en saturation de l'air et le milieu de culture comprend un condensat de polyoxyproylène/polyoxyéthylène et le cas échéant un ou plusieurs autres agents protecteurs non toxiques.

6. Procédé selon la revendication 5 qui est en outre défini par la (les) caractéristique(s) de la revendication 2, 3 et/ou la revendication 4.

7. Milieu pour culture aérée de cellules d'insectes qui comprend des cellules d'insectes, un milieu de base et un ou plusieurs agents protecteurs comprenant au moins un condensat polyoxypropylène/polyoxyéthylène.

8. Milieu selon la revendication 7 qui est substantiellement débarrassé de composants du sérum et qui comprend en outre un composant lipidique et un composant peptone.

9. Utilisation d'une culture de cellules d'insectes obtenue selon le procédé de culture selon l'une quelconque des revendications 1 à 6 pour la production d'un produit recombinant ou viral.

10. Utilisation dans un milieu de culture d'un condensat polyoxypropylène/polyoxyéthylène à titre d'agent protecteur pour la culture par aspersion de cellules d'insectes pour protéger lesdites cellules contre toute dégradation ou mort.

11. Utilisation selon la revendication 10 qui comprend en outre les caractéristiques de la revendication 7 et/ou de la revendication 8.

12. Utilisation selon la revendication 10 ou la revendication 11, selon laquelle le milieu de culture est un milieu de culture aéré.

13. Procédé pour la production d'un produit viral ou recombinant en utilisant des cellules d'insectes, qui comprend l'utilisation d'un condensat de polyoxypropylène/polyoxyéthylène à titre d'agent protecteur dans un milieu de culture par aspersion utilisé pour la culture des cellules, le procédé comprenant en outre le cas échéant la (les) caractéristique(s) spécifique(s) de l'une ou plusieurs des revendications 2 à 6.

14. Procédé selon la revendication 13, selon lequel les cellules sont cultivées par culture aérée.
